# EUROPÄISCHE PATENTANMELDUNG

(11) **EP 3 489 571 A1**
(43) Veröffentlichungstag der Anmeldung: **29.05.2019**
(21) Anmeldenummer: 17203463.9
(22) Anmeldetag: 24.11.2017
(51) Int. Cl.: F21K 9/23, A61N 5/06, F21Y 113/13, F21V 3/02, F21V 3/04, F21W 131/20, F21Y 115/10

(54) **LICHTTHERAPIELAMPE UND THERAPIEHANDGERÄT MIT EINER SOLCHEN**

(71) Anmelder: Wolff, Friedrich, 4125 Riehen (CH)
(72) Erfinder: Wolff, Friedrich, 4125 Riehen (CH)
(74) Vertreter: Latscha Schöllhorn Partner AG

(57) **Zusammenfassung**

Eine Lichttherapielampe (1) umfasst eine erste Gruppe von Leuchtdioden (32), die dazu ausgebildet sind, Licht in einem schmalbandigen ersten Wellenspektrum abzustrahlen, wobei das erste Wellenspektrum eine erste Spitze aufweist, die in einem Bereich von etwa 390 Nanometer bis etwa 410 Nanometer liegt; eine zweite Gruppe von Leuchtdioden (31), die dazu ausgebildet sind, Licht in einem schmalbandigen zweiten Wellenspektrum abzustrahlen, wobei das zweite Wellenspektrum eine zweite Spitze aufweist, die etwa die doppelte Wellenlänge der ersten Spitze aufweist; und eine Optik (2), welche die erste Gruppe von Leuchtdioden (32) und die weiten Gruppe von Leuchtdioden (31) nach aussen hin abdeckt, so dass von der ersten Gruppe von Leuchtdioden (32) und von der zweiten Gruppe von Leuchtdioden (31) abgestrahltes Licht die Optik (2) durchdringt.

## Beschreibung

### Technisches Gebiet

Die Erfindung betrifft einen Lampe mit einem Leuchtmittel, das Licht in einer zur Therapie von verschiedenen Indikationen geeigneten Qualität abgeben kann, sowie ein Therapiehandgerät mit einer solchen Lampe.

### Stand der Technik

Zur Therapie verschiedener krankheits-, alters- und belastungsbedingter Symptome und Ursachen ist der Einsatz beziehungsweise die Applikation von Licht bekannt. Dabei ist für viele Anwendungen die Qualität und Intensität des applizierten Lichtes von Bedeutung. Beispielsweise wird bei gewissen Andendungen wie beispielsweise zur Reduktion und zum Abbau von Hautfalten angestrebt, die Kollagensynthese in der Haut mit Licht anzuregen. Beispielsweise ist aus der US 2013/0190844 A1 ein Gerät bekannt, das mittels unterschiedlicher LED erzeugtes gelbes Licht mit rotem Licht mischt und dies auf die Haut eines Patienten appliziert.

Auch ist es bekannt, energiereicheres Licht kürzerer Wellenlänge auf die Haut zu applizieren. Beispielsweise wird zur Behandlung von entzündlichen Hautkrankheiten wie beispielsweise Neurodermitis blaues Licht auf die Haut appliziert.

Typischerweise ist jedoch die Eindringtiefe von kurzwelligem Licht kleiner als von langwelligem Licht. Zudem findet beim Durchdringen oberer Hautschichten in untere Hautschichten eine Veränderung der Lichtqualität beispielsweise in der Form von einer Rotverschiebung statt.

Entsprechend ist es in der herkömmlichen Lichttherapie häufig schwierig oder auch nicht möglich, Licht beziehungsweise elektromagnetische Strahlung in einer bestimmten vordefinierten Qualität beziehungsweise Wellenlänge an einem Zielort in der Haut insbesondere in unteren Hautschichten zu applizieren.

Vor diesem Hintergrund ist es eine Aufgabe der vorliegenden Erfindung eine Vorrichtung vorzuschlagen, die es ermöglicht, Licht in einer bestimmten Qualität und beispielsweise verhältnismässig kurzwelliges Licht wie violettes oder blaues Licht in untere Hautschichten zu applizieren.

### Darstellung der Erfindung

Die Aufgabe wird erfindungsgemäss durch eine Lichttherapielampe gelöst, wie sie im unabhängigen Anspruch 1 definiert ist, sowie durch ein Therapiehandgerät, wie es im unabhängigen Anspruch 14 definiert ist. Vorteilhafte Ausführungsvarianten der Erfindung ergeben sich aus den abhängigen Ansprüchen.

Das Wesen der Erfindung besteht im Folgenden: Line Lichttherapielampe umfasst eine erste Gruppe von Leuchtdioden, eine zweite Gruppe von Leuchtdioden und eine Optik. Die Leuchtdioden der ersten Gruppe beziehungsweise die ersten Leuchtdioden sind dazu ausgebildet, Licht in einem schmalbandigen ersten Wellenspektrum abzustrahlen. Das erste Wellenspektrum weist eine erste Spitze auf, die in einem Bereich von etwa 390 Nanometer (nm) bis etwa 410 nm liegt. Die Leuchtdioden der zweiten Gruppe beziehungsweise die zweiten Leuchtdioden sind dazu ausgebildet, Licht in einem schmalbandigen zweiten Wellenspektrum abzustrahlen. Das zweite Wellenspektrum hat eine zweite Spitze, die etwa die doppelte Wellenlänge der ersten Spitze aufweist. Die Optik deckt die Leuchtdioden der ersten Gruppe und der zweiten Gruppe nach aussen hin ab, so dass von der ersten Gruppe von Leuchtdioden und von der zweiten Gruppe von Leuchtdioden abgestrahltes Licht die Optik durchdringt.

Der Begriff "Leuchtdiode" wird Zusammenhang mit der Erfindung synonym zum weit verbreiteten englischen Begriff "Light Emitting Diode" oder dem Akronym "LED" verwendet. Unter einer Leuchtdiode beziehungsweise lichtemittierenden Diode oder auch Lumineszenz-Diode kann ein ein lichtemittierendes Halbleiter-Bauelement verstanden werden, dessen elektrische Eigenschaften einer Diode entsprechen. Fliesst durch diese Diode elektrischer Strom, so strahlt sie Licht, Infrarotstrahlung oder auch Ultraviolettstrahlung mit einer vom Halbleitermaterial und der Dotierung abhängigen Wellenlänge ab. LED können in einer Aufsicht beispielsweise rund, elliptisch, quadratisch oder rechteckig ausgestaltet sein.

Die LED der ersten Gruppe von Leuchtdioden werden aufgrund ihres Wellenlängenspektrums hier auch blaue LED genannt. Analog dazu werden die LED der zweiten Gruppe von Leuchtdioden aufgrund ihres Wellenlängenspektrums hier auch violette LED genannt.

Die erfindungsgemässe Anordnung der ersten und zweiten Gruppen von Leuchtdioden ermöglicht, dass sich die roten und violetten LED in ihrem Strahlengang überschneiden. Dabei stehen die roten und violetten Strahlen der LED in einem harmonischen Verhältnis zueinander. Insbesondere passen die Wellen der violetten Strahlen beziehungsweise die violetten Wellen doppelt in die Wellen der roten Strahlen beziehungsweise die roten Wellen und bilden so die harmonische Konstellation. Ein Ergebnis des harmonischen Strahlengangs kann eine Leistungssteigerung sein. Beispielsweise wurde eine solche Leistungssteigerung von ca. 30% festgestellt, die sich hauptsächlich im violetten Bereich manifestierte.

Bekanntlich werden bei therapeutischen Applikationen von Bereiche des sichtbaren Rots und einen Anteil des Infrarots A zu einer wirksamen Strahlenqualität zusammenfassendem Ultrarotlicht relativ schwache beziehungsweise kleine Energiemengen pro Photon transportiert. Das Ultrarotlicht verursacht aber auch kaum ein Risiko für Zellschäden und liefert dennoch eine zweckmässige Menge an wirksamer Energie aufgrund seiner vergleichsweise grossen Eindringtiefe.

Die violetten Strahlen beziehungsweise die Strahlung der violetten LED bringen nicht nur technische Vorteile, sondern haben ebenfalls vielfältige, biopositive Effekte. So ist die entgiftende Wirkung bekannt und wird bei Kleinkindern nach der Geburt zur Vorbeugung oder Behandlung von Gelbsucht eingesetzt. Andere Versuche haben einen um etwa 30% rascheren Abbau von Alkohol im Blut gezeigt. Auch ist die therapeutische Anwendung zur Behandlung von Hautkrankheiten beziehungsweise Hautstörungen wie beispielsweise Akne bekannt.

Mit der erfindungsgemässen Lichttherapielampe können die vorstehenden Effekte unterschiedlicher Strahlungen gezielt kombiniert werden. Wirkungen der Lichttherapielampe können die Förderung der Durchblutung sein, die Linderung von Schmerzen verschiedener Art wie beispielsweise von Rückenschmerzen und die Verbesserung der Hautreinheit. Dabei ist zu berücksichtigen, dass jede Wirkung von drei Kriterien abhängt: (i) Eindringtiefe, da die Strahlen Zielzelles erreichen müssen, damit sie wirken können; (ii) Absorption, die Energie der Strahlen muss zu den Atomen beziehungsweise Molekülen passen, um aufgenommen werden zu können; und (iii) Dosis, die Intensität der Strahlung muss eine bestimmte Energie enthalten, um die Schwelle einer therapeutischen oder anderen Reaktion zu überwinden.

Typischerweise dringen violetten Strahlen bis in die Dermis vor und erreichen dort Blutgefässe und Nervenenden. Insofern kann die violette Strahlung der roten Strahlung überlegen sein, weil die rote Strahlung sich in tieferen Schichten der Haut verlieren kann, wohingegen die violette Strahlung ihre Energie zielgerecht anliefern kann. Die Absorption der eingestrahlten Photonen kann je nach Energiegehalt, eine fotochemische Reaktion oder die Öffnung von Wasserstoffbrücken bewirken. Letzteres kann reversibel sein. Dabei kann die rote Strahlung solche Zugbrücken öffnen dank ihrer geringeren Energie. Die violette Strahlung ist dazu zu energiereich. Im Gegenzug kann die violette Strahlung die Fähigkeit haben Stickstoffmonoxid (NO) zu bilden, das Blutgefässe erweitern kann. Die violette Strahlung kann den Blutfluss also nicht durch Wärme erhöhen, sondern durch die Bindung von NO. NO kann auch an Schmerzmodulation beteiligt sein. Die erfindungsgemässe harmonische Kombination von roter und violetter Strahlung kann ermöglichen, dass die violette Strahlung tiefer in die Haut eindringen kann und quasi von der roten Strahlung mitgenommen beziehungsweise transportiert wird.

Medizinische beziehungsweise krankhafte Indikationen, bei denen die Lichttherapielampe eingesetzt werden kann, können sein: Arthralgie (Gelenkschmerzen); Arthrose (Gelenkverschleiss); Periathropathia humeroscapularis (verschiedene degenerative Prozesse im Bereich der Rotatorenmanschette, der Gelenkkapsel oder der Bizepssehne am Schultergelenk); entzündlichen Affektionen im Hals-Nasen-Ohren-Bereich (HNO) wie beispielsweise eine Sinusitis (Nasennebenhöhlenentzündung); Fibrositis-Syndrom (teilweise als "Weichteilrheumatismus" bezeichnete schmerzhaften Zustände beziehungsweise krankhafte Vorgänge in den Muskeln (Myositis) und bindegewebsreichen Strukturen der Skelettweichteile (Bursitis, Fasziitis, Periostitis, Periarthritis, Tendinitis, Tendovaginitis), Nervenscheiden (Neuritis) etc.); Fibromyalgie-Syndrom (Syndrom, welches zu Schmerzen im gesamten Muskel- und Skelett-System führt und zusätzlich noch durch Steifigkeit, Empfindungsstörungen, Schlafstörungen und chronische Erschöpfung gekennzeichnet ist); Hautaffektion; Myalgie (diffuser oder lokalisierter Muskelschmerz, der beispielsweise als Muskelkater oder in Kombination mit einer Verspannung auftritt); Myogelosen (knotenartige oder wulstförmige, klar umschriebene Verhärtungen in der Muskulatur); Myotendopathien (schmerzhafte Erkrankung der Muskelansatzsehnen); Neuritiden (Nervenentzündung); rheumatische Gelenkaffektion; und schmerzhafte Wirbelsäulenaffektion (beispielsweise bei degenerativen Veränderungen oder in Form eines Wurzelreizsyndroms wie eine Reizung einer Nervenwurzel beispielsweise durch mechanische Kompression).

Neben den vorstehenden Indikation kann die Lichttherapielampe auch zur kosmetischen Therapie eingesetzt werden, beispielsweise zur: Verbesserung der Hautbeschaffenheit (beispielsweise Festigung von geschwächtem Bindegewebe durch Aktivierung von Collagen); und photodynamischen Therapie (PDT). Die PDT gilt als vergleichsweise unkomplizierte und schonende Therapiemöglichkeit zur effektiven Behandlung von Hauttumoren. Ein typisches Krankheitsbild stellt die aktinische Keratose dar. Die PDT kann eine Alternative zu invasiven Therapien und Verfahren darstellen.

Vorzugsweise umfasst die Lichttherapielampe eine Platine, auf der die erste Gruppe von LED und die zweite Gruppe von LED in einer Ebene montiert sind. Der Begriff "Platine" kann sich in diesem Zusammenhang auf eine Leiterplatte (Printed Circuit Board, PCB) beziehen, die ein Träger für elektronische Bauteile ist. Im allgemeinen dienen Platinen der mechanischen Befestigung und elektrischen Verbindung elektronischen Bauteile. Üblicherweise bestehen Leiterplatten beziehungsweise Platinen aus einem elektrisch isolierendem Material mit daran haftenden, leitenden Verbindungen (Leiterbahnen). Als isolierendes Material ist faserverstärkter Kunststoff gängig. Die Leiterbahnen werden zumeist aus einer dünnen Schicht Kupfer geätzt. Die Bauelemente werden auf Lötflächen (Pads) oder in Lötaugen gelötet. Grössere Komponenten können auch mit Kabelbindern, Klebstoff oder Verschraubungen auf der Platine befestigt werden. Neben den roten und violetten LED kann die Platine zusätzlich mit einer Steuerungselektronik zum Betreiben der LED ausgestattet sein.

Eine solche Platine ermöglicht ein effizientes Betreiben und Herstellen der LED. Zudem kann die Anordnung in einer Ebene ein effizientes Mischen des Lichts zu einer harmonisch abgestimmten Strahlung ermöglichen.

Das schmalbandige erste Wellenspektrum und das schmalbandige zweite Wellenspektrum können jeweils eine Breite von maximal etwa 40 nm aufweisen. Beispielsweise kann sich also das erste Wellenspektrum von 380 nm bis 410 nm erstrecken und eine Spitze bei 395 nm aufweisen. Ein solches schmalbandiges Spektrum kann die Bereitstellung von einer spezifischen Lichtqualität ermöglichen, die sich effizient harmonisch mischen lässt.

Vorzugsweise ist die Optik dazu ausgestaltet, von der ersten Gruppe von LED abgestrahltes Licht und von der zweiten Gruppe von LED abgestrahltes Licht zu mischen. Dabei ist die Optik vorzugsweise als Diffusor ausgebildet. Eine solche Optik kann das Mischen der roten und der violetten Strahlungen verbessern oder gar erst ermöglichen.

Bevorzugt weist die Optik einen kugelsegmentförmigen Abschnitt auf. Auf diese Weise kann einfach ein gleichmässig beleuchteter Strahlenaustritt erreicht werden. Die Kugelsegmentform kann zudem eine gleichmässige Bestrahlungsstärke auf einer Körperoberfläche ermöglichen.

Dabei entspricht die Ebene, auf der die erste Gruppe von LED und die zweite Gruppe von LED montiert sind, vorzugsweise einer Äquatorebene des kugelsegmentförmigen Abschnitts der Optik.

Vorzugsweise umfasst die Lichttherapielampe eine Gehäusebasis, an der die Optik montiert ist, sodass die Gehäusebasis und die Optik die erste Gruppe von LED und die zweite Gruppe von LED umschliessen. Eine solche Gehäusebasis ermöglicht eine effiziente und verhältnismässig einfach handhabbare Ausgestaltung der Lichttherapielampe. Dabei umfasst die Lichttherapielampe vorzugsweise weiter einen wärmeleitenden Flanschring, der von der Gehäusebasis beziehungsweise der Optik radial absteht und insbesondere an die erste Gruppe von Leuchtdioden und die zweite Gruppe von Leuchtdioden thermisch gekoppelt ist. Dabei liegt der Flanschring vorzugsweise im Wesentlichen in der Äquatorebene. Mit einem solchen Flanschring kann auf effiziente Weise Wärme von den LED abgeleitet werden. Insbesondere kann mit einem solchen Flanschring verhindert werden, dass die Lichttherapielampe aktiv beispielsweise mittels Ventilation gekühlt werden muss.

Zudem weist die Lichttherapielampe bevorzugt einen passiven Kühlkörper auf, der in der Gehäusebasis angeordnet ist und an den die erste Gruppe von Leuchtdioden und die zweite Gruppe von Leuchtdioden thermisch gekoppelt sind. Insbesondere zusammen mit dem Flanschring kann dadurch eine ausreichende und effiziente Kühlung erreicht werden, ohne dass die Lichttherapielampe überhitzt oder an einer Aussenfläche zu heiss wird. Das kann insbesondere von Bedeutung sein, wenn verhältnismässig leistungsstarke LED eingesetzt werden, wie dies bei vielen Anwendungen gewünscht ist.

Bevorzugt weist die Lichttherapielampe einen Sockel auf, der vorzugsweise als Edisonsockel Typ 27 beziehungsweise E-27-Sockel ausgebildet ist. Ein solcher Sockel kann in bekannter Weise in eine Fassung montiert und über diese mit Strom gespeist werden.

Vorzugsweise ist die Lichttherapielampe, Licht mit einer Strahlungsintensität in einem Bereich von zwischen etwa 20 Watt (W) bis etwa 40 W, in einem Bereich von etwa 25 W bis etwa 35 W oder von etwa 30 W abzustrahlen. Eine solche Ausgestaltung kann insbesondere die Verwendung diesbezüglich ausgebildeter erste LED und zweite LED mit sich bringen. Mit einer solchen Lichttherapielampe kann eine Beleuchtungsstärke von etwa 15000 Lux beziehungsweise ein Lichtstrom von etwa 3000 Lumen bei einem Abstand von etwa 10 cm erreicht werden. Dies kann für viele Anwendungen bevorzugt sein.

Ein anderer Aspekt der Erfindung betrifft ein Therapiehandgerät, das eine Lichttherapielampe wie vorstehend beschrieben und einen Griff umfasst. Mit einem solchen Handtherapiegerät können die oben beschriebenen Effekte und Vorteile der erfindungsgemässen Lichttherapielampe und derer bevorzugten Ausführungsformen effizient erreicht werden. Insbesondere kann die Anwendung auf einfache Weise manuell erfolgen.

### Kurze Beschreibung der Zeichnungen

Weitere vorteilhafte Ausgestaltungen der Erfindung ergeben sich aus der nachfolgenden Beschreibung von Ausführungsbeispielen der Erfindung mit Hilfe der schematischen Zeichnung. Insbesondere wird im Folgenden die erfindungsgemässe Lichttherapielampe unter Bezugnahme auf die beigefügten Zeichnungen anhand von Ausführungsbeispielen detaillierter beschrieben. Es zeigen:
- Fig. 1: eine schematische Seitenansicht eines Ausführungsbeispiels einer erfindungsgemässen Lichttherapielampe;
- Fig. 2: eine schematische perspektivische Explosionsansicht der Lichttherapielampe von Fig. 1; und
- Fig. 3: eine Illustration der harmonischen Kombination von roten und violetten Lichtstrahlen.

### Weg(e) zur Ausführung der Erfindung

Bestimmte Ausdrücke werden in der folgenden Beschreibung aus praktischen Gründen verwendet und sind nicht einschränkend zu verstehen. Die Wörter "rechts", "links", "unten" und "oben" bezeichnen Richtungen in der Zeichnung, auf die Bezug genommen wird. Die Ausdrücke "nach innen", "nach aussen" "unterhalb", "oberhalb", "links", "rechts" oder ähnliche werden zur Beschreibung der Anordnung bezeichneter Teile zueinander, der Bewegung bezeichneter Teile zueinander und der Richtungen hin zum oder weg vom geometrischen Mittelpunkt der Erfindung sowie benannter Teile derselben wie in den Fig. dargestellt verwendet. Diese räumlichen Relativangaben umfassen auch andere Positionen und Ausrichtungen als die in den Fig. dargestellten. Zum Beispiel wenn ein in den Fig. dargestelltes Teil umgedreht wird, sind Elemente oder Merkmale, die als "unterhalb" beschrieben sind, dann "oberhalb". Die Terminologie umfasst die oben ausdrücklich erwähnten Wörter, Ableitungen von denselben und Wörter ähnlicher Bedeutung.

Um Wiederholungen in den Fig. und der zugehörigen Beschreibung der verschiedenen Aspekte und Ausführungsbeispiele zu vermeiden, sollen bestimmte Merkmale als gemeinsam für verschieden Aspekte und Ausführungsbeispiele verstanden werden. Das Weglassen eines Aspekts in der Beschreibung oder einer Fig. lässt nicht darauf schliessen, dass dieser Aspekt in dem zugehörigen Ausführungsbeispiel fehlt. Vielmehr kann ein solches Weglassen der Klarheit und dem Verhindern von Wiederholungen dienen. In diesem Zusammenhang gilt für die gesamte weitere Beschreibung folgende Festlegung: Sind in einer Figur zum Zweck zeichnerischer Eindeutigkeit Bezugszeichen enthalten, aber im unmittelbar zugehörigen Beschreibungstext nicht erwähnt, so wird auf deren Erläuterung in vorangehenden Figurenbeschreibungen Bezug genommen. Sind ausserdem im unmittelbar zu einer Figur gehörigen Beschreibungstext Bezugszeichen erwähnt, die in der zugehörigen Figur nicht enthalten sind, so wird auf die vorangehenden und nachstehenden Figuren verwiesen. Ähnliche Bezugszeichen in zwei oder mehreren Fig. stehen für ähnliche oder gleiche Elemente.

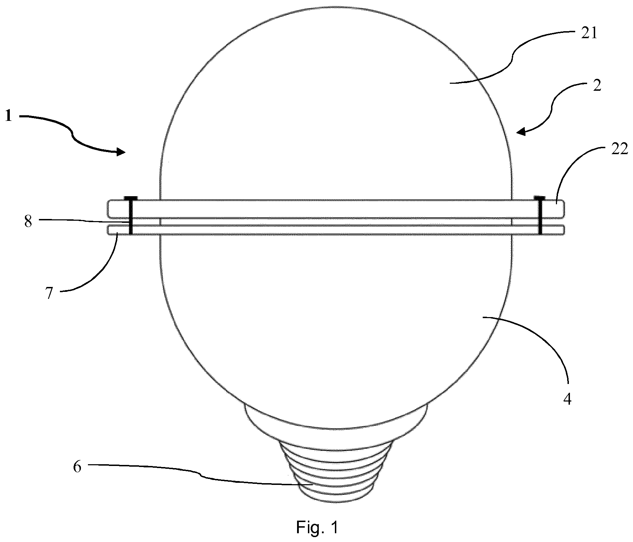

Fig. 1 zeigt ein Ausführungsbeispiel einer erfindungsgemässen Lichttherapielampe 1 mit einer quasi halbkugelförmigen Gehäusebasis 4 und einer Optik 2. Die Optik 2 ist einteilig aus einem diffus transparenten beziehungsweise zumindest teilweise lichtdurchlässigen sandgestrahlten Plexiglas hergestellt. Sie weist einen nach oben endenden halbkugelförmigen Abschnitt 21 und einen radial nach aussen abstehenden Montageflansch 22 auf.

Die Gehäusebasis 4 ist aus Aluminium hergestellt. Auf sie drauf ist ein radial abstehender und parallel zum Montageflansch 22 der Optik 2 ausgerichteter Flanschring 7 in Passform aufgeklemmt. Der Flanschring 7 und die Gehäusebasis 4 bilden so zusammen eine Einheit. Die Optik 2 ist an der Gehäusebasis 4 befestigt, indem der Montageflansch 22 mittels vier Schrauben 8 an den Flanschring 7 geschraubt ist.

Die Lichttherapielampe 1 umfasst weiter einen E27-Sockel 6, der sich von der Gehäusebasis 4 nach unten erstreckt. Der E27-Sockel 6 ist mit einem Gewinde ausgestattet, über das er in eine passenden E27-Fassung eingedreht werden kann, so dass die Lichttherapielampe 1 von der E27-Fassung gehalten und über diese mit Strom versorgt ist.

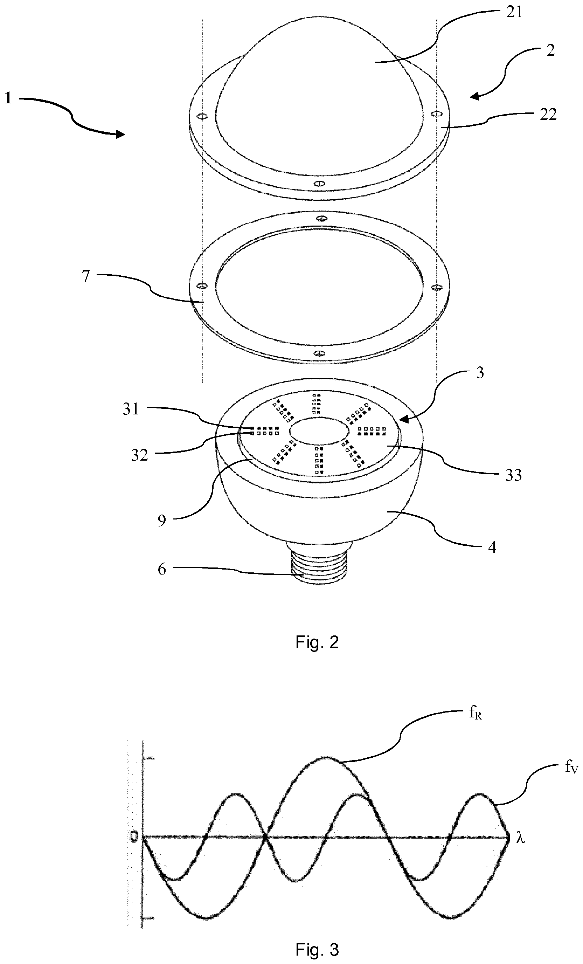

Wie in Fig. 2 ersichtlich ist im Innern der Lichttherapielampe 1 ein Leuchtmittel 3 angeordnet. Das Leuchtmittel 3 umfasst eine in einer Äquatorebene des halbkugelförmigen Abschnitts 21 der Optik liegende Platine 33, die an ihrer der Optik zugewandten Oberseite mit roten LED 31 und violetten LED 32 bestückt. Die violetten LED 32 sind in einer ersten Gruppe von acht radial und sternförmig angeordneten Reihen von jeweils fünf violetten LED 32 angeordnet. Die roten LED 31 sind in einer zweiten Gruppe von acht Reihen angeordnet, die parallel zu den Reihen der violetten LED 32 liegen. Jeweils zwei benachbarte rote und violette LED 31, 32 bilden zusammen ein Paar. Die violetten LED 32 weisen ein schmalbandiges beziehungsweise 20 nm breites erstes Wellenspektrum mit einer ersten Spitze bei 400 nm auf. Die roten LED 32 weisen ein schmalbandiges beziehungsweise 20 nm breites zweites Wellenspektrum mit einer zweiten Spitze bei 800 nm auf.

Unterhalb der Platine 33 ist ein Kühlkörper 9 angeordnet, der sich in die Gehäusebasis 4 hinein erstreckt. Er weist zahlreiche Lamellen auf, über die ein verhältnismässig grosser Wärmeaustausch möglich ist. Der Kühlkörper 9 und der Flanschring 7 sind thermisch an die LED 31, 32 gekoppelt und ermöglichen zusammen eine effiziente passive Kühlung.

Fig. 3 zeigt die harmonische Mischung der von den violetten LED 32 und den roten LED 31 abgegebenen Strahlung. Insbesondere haben die violetten Wellen f_{V} die halbe Wellenlänge der roten Wellen f_{R}, sodass jeweils harmonisch zwei violette Wellen f_{V} in eine rote Welle f_{R} eingebettet sind.

Obwohl die Erfindung mittels der Figuren und der zugehörigen Beschreibung dargestellt und detailliert beschrieben ist, sind diese Darstellung und diese detaillierte Beschreibung illustrativ und beispielhaft zu verstehen und nicht als die Erfindung einschränkend. Um die Erfindung nicht zu verklären, können in gewissen Fällen wohlbekannte Strukturen und Techniken nicht im Detail gezeigt und beschrieben sein. Es versteht sich, dass Fachleute Änderungen und Abwandlungen machen können, ohne den Umfang der folgenden Ansprüche zu verlassen. Insbesondere deckt die vorliegende Erfindung weitere Ausführungsbeispiele mit irgendwelchen Kombinationen von Merkmalen ab, die von den explizit beschriebenen Merkmalskombinationen abweichen können.

Die vorliegende Offenbarung umfasst auch Ausführungsformen mit jeglicher Kombination von Merkmalen, die vorstehend oder nachfolgend zu verschiedenen Ausführungsformen genannt oder gezeigt sind. Sie umfasst ebenfalls einzelne Merkmale in den Figuren, auch wenn sie dort im Zusammenhang mit anderen Merkmalen gezeigt sind und/oder vorstehend oder nachfolgend nicht genannt sind. Auch können die in den Figuren und der Beschreibung beschriebenen Alternativen von Ausführungsformen und einzelne Alternativen deren Merkmale vom Erfindungsgegenstand beziehungsweise von den offenbarten Gegenständen ausgeschlossen sein. Die Offenbarung umfasst Ausführungsformen, die ausschliesslich die in den Ansprüchen beziehungsweise in den Ausführungsbeispielen beschriebenen Merkmale umfasst sowie auch solche, die zusätzliche andere Merkmale umfassen.

Im Weiteren schliesst der Ausdruck "umfassen" und Ableitungen davon andere Elemente oder Schritte nicht aus. Ebenfalls schliesst der unbestimmte Artikel "ein" bzw. "eine" und Ableitungen davon eine Vielzahl nicht aus. Die Funktionen mehrerer in den Ansprüchen aufgeführter Merkmale können durch eine Einheit beziehungsweise einen Schritt erfüllt sein. Die Begriffe "im Wesentlichen", "etwa", "ungefähr" und dergleichen in Verbindung mit einer Eigenschaft beziehungsweise einem Wert definieren insbesondere auch genau die Eigenschaft beziehungsweise genau den Wert. Die Begriffe "etwa" und "ungefähr" im Zusammenhang mit einem gegebenen Zahlenwert oder -bereich kann sich auf einen Wert beziehungsweise Bereich beziehen, der innerhalb 20%, innerhalb 10%, innerhalb 5% oder innerhalb 2% des gegebenen Werts beziehungsweise Bereichs liegt.

## Patentansprüche

1. Lichttherapielampe (1) mit
einer ersten Gruppe von Leuchtdioden (32), die dazu ausgebildet sind, Licht in einem schmalbandigen ersten Wellenspektrum abzustrahlen, wobei das erste Wellenspektrum eine erste Spitze aufweist, die in einem Bereich von etwa 390 Nanometer bis etwa 410 Nanometer liegt;
einer zweiten Gruppe von Leuchtdioden (31), die dazu ausgebildet sind, Licht in einem schmalbandigen zweiten Wellenspektrum abzustrahlen, wobei das zweite Wellenspektrum eine zweite Spitze aufweist, die etwa die doppelte Wellenlänge der ersten Spitze aufweist; und
einer Optik (2), welche die erste Gruppe von Leuchtdioden (32) und die weiten Gruppe von Leuchtdioden (31) nach aussen hin abdeckt, so dass von der ersten Gruppe von Leuchtdioden (32) und von der zweiten Gruppe von Leuchtdioden (31) abgestrahltes Licht die Optik (2) durchdringt.

2. Lichttherapielampe (1) nach Anspruch 1, die eine Platine (33) aufweist, auf der die erste Gruppe von Leuchtdioden (32) und die weiten Gruppe von Leuchtdioden (31) in einer Ebene montiert sind.

3. Lichttherapielampe (1) nach Anspruch 1 oder 2, bei der das schmalbandige erste Wellenspektrum und das schmalbandige zweite Wellenspektrum jeweils eine Breite von maximal etwa 40 Nanometer aufweisen.

4. Lichttherapielampe (1) nach einem der vorangehenden Ansprüche, bei der die Optik (2) dazu ausgestaltet ist, von der ersten Gruppe von Leuchtdioden (32) abgestrahltes Licht und von der zweiten Gruppe von Leuchtdioden (31) abgestrahltes Licht zu mischen.

5. Lichttherapielampe (1) nach Anspruch 4, bei der die Optik (2) als Diffusor ausgebildet ist.

6. Lichttherapielampe (1) nach einem der vorangehenden Ansprüche, bei der die Optik (2) einen kugelsegmentförmigen Abschnitt (21) aufweist.

7. Lichttherapielampe (1) nach Anspruch 2 und 6, bei der die Ebene, auf der die erste Gruppe von Leuchtdioden (32) und die weiten Gruppe von Leuchtdioden (31) montiert sind, einer Äquatorebene des kugelsegmentförmigen Abschnitts (21) der Optik (2) entspricht.

8. Lichttherapielampe (1) nach einem der vorangehenden Ansprüche, die eine Gehäusebasis (4) umfasst, an der die Optik (2) montiert ist, sodass die Gehäusebasis (4) und die Optik (2) die erste Gruppe von Leuchtdioden (32) und die weiten Gruppe von Leuchtdioden (31) umschliessen.

9. Lichttherapielampe (1) nach Anspruch 8, die einen wärmeleitenden Flanschring (7) umfasst, der von der Gehäusebasis (4) beziehungsweise der Optik (2) radial absteht.

10. Lichttherapielampe (1) nach Anspruch 7 und 9, bei dem der Flanschring (7) im Wesentlichen in der Äquatorebene liegt.

11. Lichttherapielampe (1) nach Anspruch 8 oder 9, die einen passiven Kühlkörper (9) aufweist, der in der Gehäusebasis (4) angeordnet ist und an den die erste Gruppe von Leuchtdioden (32) und die zweite Gruppe von Leuchtdioden (31) thermisch gekoppelt sind.

12. Lichttherapielampe (1) nach einem der vorangehenden Ansprüche, die einen Sockel (6) und vorzugsweise eine E-27-Sockel aufweist.

13. Lichttherapielampe (1) nach einem der vorangehenden Ansprüche, die dazu ausgebildet ist, Licht mit einer Strahlungsintensität in einem Bereich von zwischen etwa 20 Watt bis etwa 40 Watt, in einem Bereich von etwa 25 Watt bis etwa 35 Watt oder von etwa 30 Watt abzustrahlen.

14. Therapiehandgerät, das eine Lichttherapielampe (1) nach einem der vorangehenden Ansprüche und einen Griff umfasst.
